(19) Europäisches Patentamt
European Patent Office
Office européen des brevets

(11) Veröffentlichungsnummer: **0 038 303**
**A2**

(12) # EUROPÄISCHE PATENTANMELDUNG

(21) Anmeldenummer: **81810138.8**

(22) Anmeldetag: **08.04.81**

(51) Int. Cl.³: **C 07 D 307/93**, A 01 N 43/12, A 01 N 53/00

(30) Priorität: **14.04.80 CH 2859/80**

(43) Veröffentlichungstag der Anmeldung: **21.10.81 Patentblatt 81/42**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Baumann, Marcus, Dr., Rührbergerstrasse 6, CH-4058 Basel (CH)**

(54) **Cyclopropandicarbonsäure-Isoimide, Verfahren zu deren Herstellung und deren Verwendung als Fungizid.**

(57) Cyclopropandicarbonsäure-isoimide der Formel

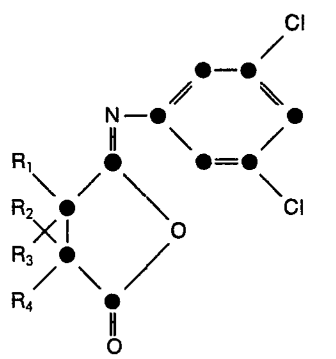

(I),

worin $R_1$, $R_2$, $R_3$ und $R_4$ unabhängig voneinander Wasserstoff oder Alkyl mit 1—4 C-Atomen bedeuten, eignen sich zur Bekämpfung verschiedenartiger Pilze und besitzen insbesondere eine ausgezeichnete Botrytis-Wirkung. Sie können durch Cyclisierung der entsprechenden Amidcarbonsäuren bei Temperaturen zwischen etwa —20° C und +100° C in Gegenwart von Dehydratisierungsmitteln, wie Acetanhydrid, N,N'-Dicyclohexylcarbodiimid oder Keten, und gegebenenfalls in Gegenwart eines inerten organischen Lösungsmittels, wie Toluol, hergestellt werden.

EP 0 038 303 A2

0038303

5-12806/ZFO/=

Cyclopropandicarbonsäure-isoimide, Verfahren zu deren Herstellung und deren Verwendung als Fungizid

Die vorliegende Erfindung betrifft neue Cyclopropandicarbonsäure-isoimide, ein Verfahren zu deren Herstellung, fungizide Mittel, die solche Verbindungen als Aktivsubstanz enthalten, sowie die Verwendung dieser Verbindungen zur Bekämpfung von Pilzen.

In der U.S. Patentschrift 3.903.090 sind gegebenenfalls am Cyclopropanring substituierte N-(3,5-Dihalogenphenyl)-cyclopropandicarbonsäureimide mit antimikrobiellen Eigenschaften, u.a. auch fungiziden Eigenschaften, beschrieben.

Die Lektüre dieser Beschreibung vermittelt dem Fachmann die Erkenntnis, dass die symmetrische Dicarbonimid-Struktur für die biologische Wirkung ein wichtiger struktureller Faktor ist. In den Zeilen 60-68 der Spalte 1 der Beschreibung wird darauf hingewiesen, dass bereits kleine strukturelle Aenderungen im Wirkstoff-Molekül die Mikrobizid-Wirkung vollständig zum Verschwinden bringen oder diese in einigen Fällen in eine Herbizid-Wirkung umwandeln können.

Es wurden nun neue Cyclopropandicarbonsäure-isoimide der Formel I

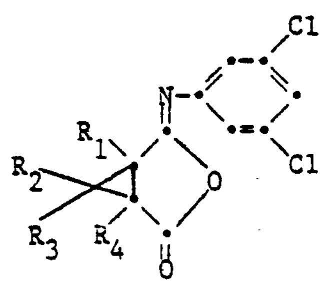

(I),

gefunden, worin $R_1$, $R_2$, $R_3$ und $R_4$ unabhängig voneinander Wasserstoff oder Alkyl mit 1-4 C-Atomen bedeuten.

Die Verbindungen der Formel I weisen ausgezeichnete fungizide Eigenschaften ohne Phytotoxizität auf und besitzen insbesondere eine ausgeprägte Botrytis-Wirkung. Botrytis spp. (B. cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar.

Die Verbindungen der Formel I besitzen ein für die praktischen Bedürfnisse sehr günstiges Wirkungsspektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, vor allem Steinobst, Zierpflanzen, Reben, Hopfen, Gurkengewächse (Gurken, Kürbis, Melonen), Solanaceen, wie Kartoffeln, Tomaten und Tabak, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae, Fusarium, Helminthosporium); Basidiomycetes, wie vor allem Rostpilze (z.B. Puccinia, Tilletia); Fungi imperfecti (z.B. Moniliales u.a., Cercospora, Sclerotinia sowie Botrytis und Piricularia) und die der Klasse der Phycomycetes angehörenden Oomycetes, wie Phytophthora oder Plasmopara. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Alkylgruppen $R_1$ bis $R_4$ können geradkettig oder verzweigt sein, sind jedoch bevorzugt geradkettig und weisen insbesondere 1 oder 2 C-Atome auf. Beispiele definitionsgemässer Alkylgruppen sind:

die Methyl-, Aethyl-, n-Propyl-, Isopropyl-, n-, sek- und tert-Butylgruppe. Besonders bevorzugt sind Verbindungen der Formel I, worin entweder $R_1$ und $R_2$ je Methyl und $R_3$ und $R_4$ je Wasserstoff oder $R_3$ und $R_4$ je Methyl und $R_1$ und $R_2$ je Wasserstoff bedeuten, oder worin $R_1$ und $R_4$ je Wasserstoff und $R_2$ und $R_3$ je Methyl bedeuten. Ganz besonders bevorzugt ist die Verbindung der Formel I, worin $R_1$ und $R_4$ je Methyl und $R_2$ und $R_3$ je Wasserstoff darstellen.

Die Verbindungen der Formel I können z.B. dadurch hergestellt werden, dass man eine Verbindung der Formel II

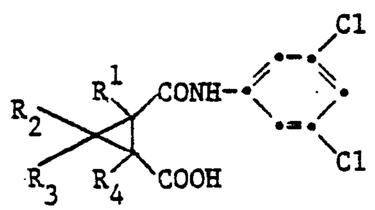

(II),

worin $R_1$, $R_2$, $R_3$ und $R_4$ die unter Formel I angegebene Bedeutung haben, bei einer Temperatur zwischen -20°C und +100°C, bevorzugt zwischen 0°C und 40°C, in Gegenwart eines Dehydratisierungsmittels und vorzugsweise in Gegenwart eines inerten organischen Lösungsmittels zum Isoimid der Formel I cyclisiert.

Isoimide sind im allgemeinen thermodynamisch labil und lagern sich relativ leicht in die entsprechenden Imide um (vgl. z.B. deutsche Offenlegungsschrift 2.715.435). Ueberraschenderweise lassen sich die Amidcarbonsäuren der Formel II unter den angegebenen Reaktionsbedingungen in stabile und als Fungizide sehr wertvolle Isoimide der Formel I überführen.

In der Beschreibung zur U.S.-Patentschrift 3.903.090 wird auf diese Möglichkeit der Isoimidbildung nicht hingewiesen, weil in dem zur Herstellung von Cyclopropandicarbonsäureimiden aus Cyclopropandicarbonsäure-monoamiden angegebenen "Standard operational process" ein Zusatz von Na-Acetat verwendet wird, der, wie man in der Zwischenzeit

weiss, automatisch bewirkt, dass bei erhöhter Temperatur von ca. 100°C keine Bildung von Isoimiden erfolgt.

Als Dehydratisierungsmittel kommen z.B. in Betracht: Anhydride von gegebenenfalls durch Halogenatome oder $C_{1-4}$-Alkylgruppen substituierten aliphatischen $C_{2-5}$-Monocarbonsäuren, wie Essigsäure-, Propionsäure-, Buttersäure- und Valeriansäureanhydrid, Trichlor-, Trifluor-, Trimethyl-, Triäthyl- und Tri-n-butylessigsäureanhydrid; Carbodiimide, wie N,N'-Diisopropylcarbodiimid und N,N'-Dicyclohexylcarbodiimid; sowie Keten. Ferner können Gemische von tertiären Aminen und den oben erwähnten Anhydriden oder Gemische von tertiären Aminen und gegebenenfalls halogenierten Acetylhalogeniden, wie Acetylchlorid, Chloracetylchlorid, Dichloracetylchlorid und Trifluoracetylchlorid, eingesetzt werden. Geeignete tertiäre Amine sind z.B. N,N-Dimethylanilin, N-Methyl-N-äthylanilin, N-Methyl- oder N-Aethyl-morpholin, Pyridin, Chinuclidin oder N,N'-Dimethylpiperazin und insbesondere Trialkylamine mit je 1-8 C-Atomen in den Alkylgruppen, wie Triäthylamin, Tri-n-butylamin, Tri-(2-äthyl-n-hexyl)-amin und Tri-n-octylamin.

Bevorzugte Dehydratisierungsmittel sind Acetanhydrid, N,N'-Dicyclohexylcarbodiimid und Keten. Dabei kann überschüssiges Acetanhydrid auch als Lösungsmittel dienen.

Geeignete inerte organische Lösungsmittel zur Durchführung der Cyclisierung sind z.B. gegebenenfalls chlorierte aromatische Kohlenwasserstoffe, wie Benzol, Toluol, Xylole und Chlorbenzol; chlorierte aliphatische Kohlenwasserstoffe, wie Chloroform, Methylenchlorid und 1,2-Dichloräthan; aliphatische und cycloaliphatische Ketone, wie Aceton, Methyläthylketon und Cyclohexanon; sowie Dialkyläther mit je 2-6 C-Atomen in den Alkylteilen und cyclische Aether, wie Tetrahydrofuran und Dioxan. Besonders bevorzugt wird die Umsetzung in Acetanhydrid oder Toluol durchgeführt.

Die Amidsäuren der Formel II sind entweder aus der US-PS 3.903.090 bekannt oder können nach an sich bekannten Methoden durch Reaktion eines durch $R_1$ bis $R_4$ substituierten Cyclopropandicarbonsäureanhydrids mit 3,5-Dichloranilin gewonnen werden.

Nach Beendigung der Umsetzung können die Isoimide der Formel I auf übliche Weise isoliert werden, z.B. indem man das Lösungsmittel abdestilliert und den Rückstand aus einem geeigneten Lösungsmittel (Methanol, Essigsäureäthylester, Aceton etc.) im Temperaturbereich des Herstellungsverfahrens umkristallisiert.

Die Verbindungen der Formel I können für sich allein oder bevorzugt zusammen mit einem geeigneten Trägermaterial und/oder Zuschlagstoffen, besonders einem oberflächenaktiven Mittel, verwendet werden. Geeignete Trägermaterialien und Zuschlagstoffe können fest oder flüssig sein und entsprechen den in der Formulierungstechnik üblichen Stoffen, wie z.B. natürlichen oder regenerierten mineralischen Stoffen, Lösungs-, Dispergier-, Netz-, Haft-, Verdickungs, Binde- oder Düngemitteln. Wirkstoffe der Formel I können im Gemisch mit anderen pestiziden oder mit pflanzenwuchsverbessernden Präparaten verwendet werden.

Der Gehalt an Wirkstoff in handelsfähigen Mitteln liegt zwischen 0,1 bis 90 Gew.-%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):
Feste Aufarbeitungsformen: Stäubemittel und Streumittel (bis zu 10 Gew.-%); Granulate, Umhüllungsgranulate, Imprägnierungsgranulate und Homogengranulate, Pellets (Körner) (1 bis 80 Gew.-%);

Flüssige Aufarbeitungsformen:

a) in Wasser dispergierbare Wirkstoffkonzentrate:
Spritzpulver (wettable powders) und Pasten (25-90 Gew.-% in der Handelspackung, 0,01 bis 15 Gew.-% in gebrauchsfertiger Lösung); Emulsions- und Lösungskonzentrate (10 bis 50 Gew.-% in der Handelspackung; 0,01 bis 15 Gew.-% in gebrauchsfertiger Lösung);

b) Lösungen (0,1 bis 20 Gew.-%); Aerosole.

Herstellungsbeispiele

Beispiel 1: 10 g 1,2-Dimethyl-N-(3',5'-dichlorphenyl)-cyclopropandicarbonsäure-monoamid (erhalten durch Umsetzung von 1,2-Dimethylcyclopropandicarbonsäureanhydrid mit 3,5-Dichloranilin) werden in 100 ml Acetanhydrid während 24 Stunden bei 20°C verrührt, wobei eine klare Lösung entsteht. Anschliessend wird das überschüssige Acetanhydrid bei 40-45°C abdestilliert, und der Rückstand wird aus Methanol kristallisiert. Man erhält 6,5 g (73% d.Th.) 1,2-Dimethyl-N-(3',5'-dichlorphenyl)-cyclopropandicarbonsäure-isoimid; Smp. 106-107°C.
IR-Spektrum (KBr): 1820 $cm^{-1}$ und 1720$cm^{-1}$.
NMR-Spektrum (60 MHz, $CDCl_3$ $\delta$ in ppm): 1,15 und 1,6 (AB-System, 2 H, J = 5 Hz, $-CH_2-$); 1,44 (s, 3H $-CH_3$(; 1,53 (s, 3H, $-CH_3$); 6,88 (m, 2 H, aromat.); 7,1 (m, 1 H, aromat).

Elementaranalyse für $C_{13}H_{11}NO_2Cl_2$ (Molgewicht 284,1):

| | | | |
|---|---|---|---|
| berechnet | C 55,0 % | H 3,9 % | Cl 25,0 % |
| gefunden | C 54,9 % | H 3,9 % | Cl 25,1 %. |

[Wirkstoff Nr. 1].

Beispiel 2: 3,0 g 1,2-Dimethyl-N-(3',5'-dichlorphenyl)-cyclopropandicarbonsäure-monoamid werden in 50 ml Toluol suspendiert. Bei 20°C wird unter Rühren während einer Stunde Keten eingeleitet, bis eine klare Lösung entsteht. Die Temperatur steigt dabei auf 26°C. Anschliessend wird das Toluol im Vakuum bei ca. 40-45°C abdestilliert,

und der Rückstand wird aus Methanol kristallisiert. Man erhält 2,0 g (72% d.Th.) 1,2-Dimethyl-N-(3',5'-dichlorphenyl)-cyclopropancarbonsäure-isoimid; Smp. 106-107°C. Die spektroskopischen Daten (IR- und NMR-Spektrum) der erhaltenen Verbindung stimmen mit denjenigen des gemäss Beispiel 1 hergestellten 1,2-Dimethyl-N-(3',5'-dichlorphenyl)-cyclopropandicarbonsäure-isoimids überein.

Beispiel 3: 3,0 g 1,2-Dimethyl-N-(3',5'-dichlorphenyl)-cyclopropandicarbonsäure-monoamid werden in 40 ml Toluol suspendiert. Unter Rühren werden auf einmal 2,2 g N,N'-Dicyclohexylcarbodiimid, gelöst in 10 ml Toluol, zugegeben. Die Temperatur des Reaktionsgemisches steigt dabei auf 35°C. Nach einer Stunde wird vom gebildeten N,N'-Dicyclohexylharnstoff abfiltriert, das Filtrat wird im Vakuum bei 40-45°C zur Trockne eingedampft, und der Rückstand wird aus Methanol kristallisiert. Man erhält 2,1 g (75% d.Th.) 1,2-Dimethyl-N-(3',5'-dichlorphenyl)-cyclopropandicarbonsäure-isoimid vom Smp. 106-107°C, dessen spektroskopische Daten mit denjenigen der gemäss Beispiel 1 erhaltenen Verbindung übereinstimmen.

Beispiel 4: Herstellung von 1,3- bzw. 2,3-Dimethyl-N-(3',5'-dichlorphenyl)-cyclopropandicarbonsäure-isoimid

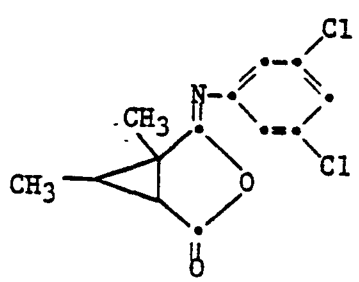

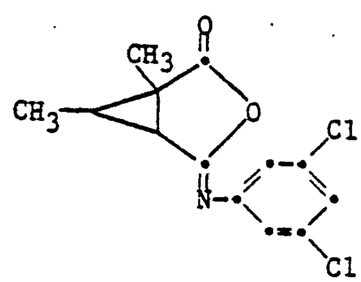

[Wirkstoff Nr. 2]

2 g 1,3- bzw. 2,3-Dimethyl-N-(3',5'-dichlorphenyl)-cyclopropandicarbonsäure-monoamid (erhalten durch Umsetzung von 1,3-Dimethylcyclopropandicarbonsäureanhydrid mit 3,5-Dichloranilin) werden in 10 ml Essigsäureanhydrid 3 Stunden bei Raumtemperatur (20-25°C) bis

zum Entstehen einer klaren Lösung verrührt. Anschliessend wird bei 40°C im Vakuum zur Trockne eingedampft, und der Rückstand wird mit Methylenchlorid über 40 g Kieselgel filtriert. Nach dem Abdampfen des Methylenchlorids werden 1,2 g des obigen Isoimid-Gemisches als farbloses Oel erhalten, das beim Abkühlen langsam kristallisiert. Ausbeute 65% d.Th.

NMR-Spektrum (100 MHz, $CDCl_3$, $\delta$ in ppm): 1,28 (d, 3H, J=6 Hz, $-CH_3$); 1,47 bzw. 1,52 (s, 3H, $-CH_3$); 1,78 (m, 1H, CH); 2,08 bzw. 2,35 (d, 1H, J=3 Hz, CH); 6,9 (m, 2H, aromat.) und 7,12 (m, 1H, aromat.).

IR-Spektrum ($CHCl_3$, $cm^{-1}$): $1850^{s}$ und $1740^{vs}$.

Elementaranalyse für $C_{13}H_{11}NO_2Cl_2$ (Molgewicht 284,1):

| | | | |
|---|---|---|---|
| berechnet | C 54,9% | H 3,9% | N 4,9% |
| gefunden | C 54,8% | H 4,1% | N 4,9% |

Beispiel 5: 2 g 1,3- bzw. 2,3-Dimethyl-N-(3',5'-dichlorphenyl)-cyclopropan-dicarbonsäure-monoamid und 1,5 g N,N'-Dicyclohexylcarbodiimid werden in 20 ml Toluol kurz auf 40°C erwärmt und dann 2 Stunden bei Raumtemperatur stehen gelassen. Anschliessend wird vom gebildeten N,N'-Dicyclohexylharnstoff abfiltriert, und das Filtrat wird im Vakuum bei 40°C zur Trockne eingedampft. Der Rückstand wird mit Methylenchlorid über 40 g Kieselgel filtriert. Nach dem Abdampfen des Lösungsmittels bei 40°C erhält man 1,6 g des im Beispiel 4 angegebenen Isoimid-Gemisches als farbloses Oel, das beim Abkühlen langsam kristallisiert. Ausbeute 85% d.Th. Die spektroskopischen Daten des erhaltenen Gemisches stimmen mit denjenigen des gemäss Beispiel 4 hergestellten Gemisches überein.

Analog zur Herstellungsmethode dieser Beispiele lassen sich auch folgende Isoimide gewinnen:

Nr. 3 N-(3',5'-dichlorphenyl)-cyclopropandicarbonsäureisoimid, Smp. 70-75°C;

Nr. 4 3,3-Dimethyl-N-(3',5'-dichlorphenyl)-cyclopropandicarbonsäureisoimid, Smp. 75-78°C;

Nr. 5 1- bzw. 2-Methyl-N-(3',5'-dichlorphenyl)-cyclopropandicarbonsäure-isoimid, Oel (IR 1670 $cm^{-1}$ [C=O]);

Nr. 6 1- bzw. 2-Aethyl-N-(3',5'-dichlorphenyl)-cyclopropandicarbonsäure-isoimid, Oel;

Nr. 7 3-Aethyl-N-(3',5'-dichlorphenyl)-cyclopropandicarbonsäure-isoimid, Smp. 78-82°C.

Die Wirkstoffe der Formel I vorliegender Erfindung können beispielsweise wie folgt formuliert werden:

Beispiel 6:

Stäubemittel: Zur Herstellung eines 5%igen Stäubemittels werden 5 Teile eines Wirkstoffes Nr. 1 bis Nr. 7 mit 95 Teilen Talkum anwendungsfertig vermahlen.

Beispiel 7

Granulat: Zur Herstellung eines 5%igen Granulates werden die folgenden Stoffe verwendet:

5 Teile Wirkstoff Nr. 1,
0,25 Teile epoxidiertes Pflanzenöl
0,25 Teile Cetylpolyglykoläther,
3,50 Teile Polyäthylenglykol
91 Teile Kaolin (Korngrösse 0,3-0,8 mm).

Die Aktivsubstanz wird mit dem epoxidierten Pflanzenöl vermischt und mit 6 Teilen Aceton gelöst; hierauf werden Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 8:

Spritzpulver: Zur Herstellung eines a) 40%igen b) 25%igen c) 10%igen Spritzpulvers werden folgende Bestandteile verwendet:

| | | |
|---|---|---|
| a) | 40 | Teile eines Wirkstoffs Nr. 1 bis Nr. 7 |
| | 5 | Teile Ligninsulfonsäure-Natriumsalz, |
| | 1 | Teil Dibutylnaphthalinsulfonsäure-Natriumsalz, |
| | 54 | Teile Kieselsäure; |
| b) | 25 | Teile eines Wirkstoffs Nr. 1 bis Nr. 7 |
| | 4,5 | Teile Calcium-Ligninsulfonat, |
| | 1,9 | Teile Champagne-Kreide/Hydroxyäthylcellulose-Gemisch (1:1), |
| | 1,5 | Teile Natriumdibutylnaphthalinsulfonat, |
| | 19,5 | Teile Kieselsäure, |
| | 19,5 | Teile Champagne-Kreide, |
| | 28,1 | Teile Kaolin; |
| c) | 10 | Teile eines Wirkstoffs Nr. 1 bis Nr. 7, |
| | 3 | Teile Gemisch der Natriumsalze von gesättigten Fettalkoholsulfaten, |
| | 5 | Teile Naphthalinsulfonsäure/Formaldehyd-Kondensat, |
| | 82 | Teile Kaolin. |

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 9

Emulgierbare Konzentrate: Zur Herstellung eines 25%igen emulgierbaren Konzentrates werden folgende Stoffe verwendet:

25 Teile eines Wirkstoffs Nr. 1 bis Nr. 7
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

Biologische Beispiele

Beispiel 10:

a) Wirkung gegen Botrytis cinerea auf Bohnen (Vicia faba)
Residual-protektive Wirkung: ca. 10 cm hohe Bohnen-Pflanzen wurden mit einer aus Spritzpulver des Wirkstoffes hergestellten Spritzbrühe besprüht, enthaltend 0,02% bzw. 0,006% Aktivsubstanz. Nach 48 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21°C erfolgte die Beurteilung des Pilzbefalls. Die Wirkstoffe Nr. 1 bis 7 erzielten in 0,02%iger Konzentration eine vollständige Verhütung des Krankheitsbefalls. Die Wirkstoffe Nr. 1 bis Nr. 5 verhüteten den Befall auch noch in 0,006%iger Konzentration.

b) Wirkung gegen Sclerotinia fructigena auf Aepfeln: Künstlich verletzte Aepfel wurden mit Sclerotinia fructigena infiziert, indem auf jede Verletzungsstelle ein Tropfen Myzelsuspension pipettiert wurde. Nach Abtrocknen des Inokulum-Tropfens wurden die Aepfel mittels einer

Spritzpulversuspension der Wirksubstanz (0,02% Aktivsubstanz) besprüht. Die behandelten Früchte wurden in Plastikbehälter gelegt und während 14 Tagen bei 20-22°C gelagert. Zur Auswertung wurde die Anzahl der angefaulten Verletzungsstellen bestimmt.

Die erfindungsgemässen Wirkstoffe Nr. 1 bis Nr. 7 verhüteten den Krankheitsbefall vollständig.

c) Wirkung gegen Monilinia auf Kirschblüten oder Pfirsichblüten (Monilinia-Befall an Steinobst): Einzelne voll erblühte Steinobstzweige werden mit einer aus einem Emulsionskonzentrat hergestellten Spritzbrühe (enthaltend 0,025% Aktivsubstanz) besprüht. Einige Stunden später werden die Blütenstände abgeschnitten, mit dem Stiel in den nassen Sand von Plastikschalen gesteckt und mit einer Sporensuspension inokuliert. Die Schalen werden dann lose mit transparenter Plastikfolie bedeckt und 2 Tage bei Raumtemperatur gehalten. Mit der Zahl von befallenen Blüten wird das Ausmass des Krankheitsbefalls bestimmt, wobei je 40 Blüten pro Wirkstoff verwendet werden. Die Verbindungen Nr. 4, 6 und 7 bewirkten eine Reduktion des Krankheitsbefalls auf unter 20%. Die Verbindungen Nr. 1, 2, 3 und 5 verhüteten den Krankheitsbefall vollständig.

Patentansprüche

1. Verbindungen der Formel I

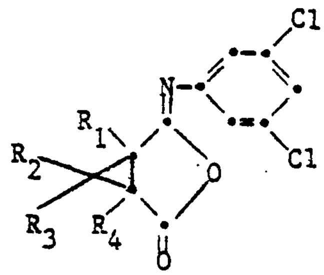

(I),

worin $R_1$, $R_2$, $R_3$ und $R_4$ unabhängig voneinander Wasserstoff oder Alkyl mit 1-4 C-Atomen darstellen.

2. Verbindungen der Formel I nach Anspruch 1, worin entweder $R_1$ und $R_2$ je Methyl und $R_3$ und $R_4$ je Wasserstoff oder $R_3$ und $R_4$ je Methyl und $R_1$ und $R_2$ je Wasserstoff bedeuten, oder worin $R_1$ und $R_4$ je Wasserstoff und $R_2$ und $R_3$ je Methyl bedeuten.

3. Verbindung der Formel I nach Anspruch 1, worin $R_1$ und $R_4$ je Methyl und $R_2$ und $R_3$ je Wasserstoff darstellen.

4. Verfahren zur Herstellung einer Verbindung der Formel I

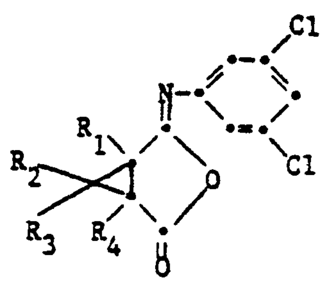

(I),

worin $R_1$, $R_2$, $R_3$ und $R_4$ unabhängig voneinander Wasserstoff oder Alkyl mit 1-4 C-Atomen bedeuten, dadurch gekennzeichnet, dass man eine Verbindung der Formel II

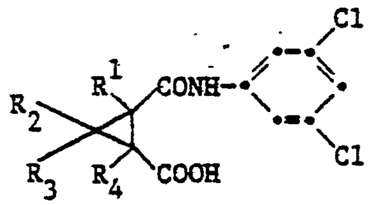

(II),

bei einer Temperatur zwischen -20°C und +100°C in Gegenwart eines Dehydratisierungsmittels zu einem Isoimid der Formel I cyclisiert.

5. Verfahren nach Anspruch 4, wobei als Dehydratisierungsmittel a) Anhydride von gegebenenfalls durch Halogenatome oder $C_{1-4}$-Alkylgruppen substituierten aliphatischen $C_{2-5}$-Monocarbonsäuren oder b) Carbodiimide oder c) Keten oder d) Gemische von gegebenenfalls halogenierten Acetylhalogeniden mit tertiären Aminen eingesetzt werden.

6. Verfahren nach Anspruch 5, dadurch gekennzeichnet, dass man als Dehydratisierungsmittel N,N'-Dicyclohexylcarbodiimid, Acetanhydrid oder Keten verwendet.

7. Verfahren nach Anspruch 4, dadurch gekennzeichnet, dass man die Cyclisierung bei einer Temperatur zwischen 0°C und 40°C vornimmt.

8. Verfahren nach Anspruch 4, dadurch gekennzeichnet, dass man die Cyclisierung in Gegenwart eines inerten organischen Lösungsmittels vornimmt.

9. Fungizides Mittel, enthaltend als Aktivsubstanz eine Verbindung der Formel I

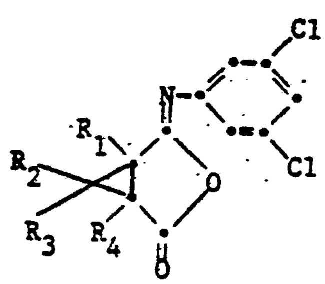

(I),

worin $R_1$, $R_2$, $R_3$ und $R_4$ unabhängig voneinander Wasserstoff oder Alkyl mit 1-4 C-Atomen bedeuten, sowie ein geeignetes Trägermaterial und/oder ein oberflächenaktives Mittel.

10. Mittel nach Anspruch 9 enthaltend eine Verbindung der Formel I, worin entweder $R_1$ und $R_2$ je Methyl und $R_3$ und $R_4$ je Wasserstoff oder $R_3$ und $R_4$ je Methyl und $R_1$ und $R_2$ je Wasserstoff bedeuten, oder worin $R_1$ und $R_4$ je Wasserstoff und $R_2$ und $R_3$ je Methyl bedeuten.

11. Mittel nach Anspruch 9 enthaltend eine Verbindung der Formel I, worin $R_1$ und $R_4$ je Methyl und $R_2$ und $R_3$ je Wasserstoff darstellen.

12. Verwendung einer Verbindung der Formel I

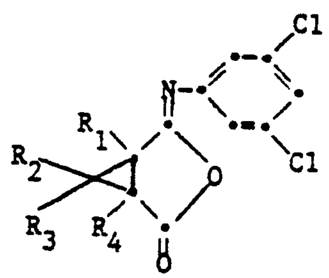

(I),

worin $R_1$, $R_2$, $R_3$ und $R_4$ unabhängig voneinander Wasserstoff oder Alkyl mit 1-4 C-Atomen bedeuten, zur Bekämpfung von Pilzen oder zur Verhütung von Pilzbefall.

13. Verwendung gemäss Anspruch 12 einer Verbindung der Formel I, worin entweder $R_1$ und $R_2$ je Methyl und $R_3$ und $R_4$ je Wasserstoff oder $R_3$ und $R_4$ je Methyl und $R_1$ und $R_2$ je Wasserstoff bedeuten, oder worin $R_1$ und $R_4$ je Wasserstoff und $R_2$ und $R_3$ je Methyl bedeuten.

14. Verwendung gemäss Anspruch 12 einer Verbindung der Formel I, worin $R_1$ und $R_4$ je Methyl und $R_2$ und $R_3$ je Wasserstoff darstellen.